# EUROPEAN PATENT SPECIFICATION

(11) **EP 1 664 344 B1**
(45) Date of publication and mention of the grant of the patent: **06.08.2008**
(21) Application number: 04769490.6
(22) Date of filing: 08.09.2004
(51) Int. Cl.: C12Q 1/68, G01N 33/68, A01K 67/027, A61P 3/04

(54) **METHOD OF DIAGNOSIS OF OBESITY**
VERFAHREN ZUR DIAGNOSE VON FETTLEIBIGKEIT
METHODE DIAGNOSTIQUE D'OBESITE

(30) Priority: 08.09.2003 EP 03292202
(43) Date of publication of application: 07.06.2006
(73) Proprietor: CENTRE NATIONAL DE LA RECHERCHE SCIENTIFIQUE (CNRS), 75016 Paris (FR)
(72) Inventor: FROGUEL, Philippe, F-93170 Bagnolet (FR); DINA, Christian, F-75009 Paris (FR); BOUTIN, Philippe, F-59200 Tourcoing (FR); SERON, Karin, F-62500 Saint-Omer (FR); CLEMENT, Karine, F-75010 Paris (FR)
(74) Representative: Warcoin, Jacques
(86) International application number: PCT/IB2004/003144
(87) International publication number: WO 2005/024063

(56) References cited:
- US-A1- 2001 044 110
- XU G MARK ET AL: "WAC, a novel WW domain-containing adapter with a coiled-coil region, is colocalized with splicing factor SC35" GENOMICS, vol. 79, no. 1, January 2002 (2002-01), pages 87-94, XP002267136 ISSN: 0888-7543 cited in the application
- WAGNER E F ET AL: "Transgenic Animals: Generation and Use" TRENDS IN GENETICS, ELSEVIER SCIENCE PUBLISHERS B.V. AMSTERDAM, NL, vol. 13, no. 12, 1 December 1997 (1997-12-01), pages 501-502, XP004098773 ISSN: 0168-9525
- BUTLER A A ET AL: "Knockout models resulting in the development of obesity" TRENDS IN GENETICS, ELSEVIER, AMSTERDAM, NL, vol. 17, no. 10, 1 October 2001 (2001-10-01), pages S50-S54, XP004307597 ISSN: 0168-9525
- CHAGNON YVON C ET AL: "The human obesity gene map: the 2002 update." OBESITY RESEARCH. UNITED STATES MAR 2003, vol. 11, no. 3, March 2003 (2003-03), pages 313-367, XP008026392 ISSN: 1071-7323
- PRICE R A ET AL: "A locus affecting obesity in human chromosome region 10p12" DIABETOLOGIA, BERLIN, DE, vol. 44, no. 3, March 2001 (2001-03), pages 363-366, XP002246820 ISSN: 0012-186X cited in the application
- IQBAL M J ET AL: "Differentially abundant mRNAs in rat liver in response to diets containing soy protein isolate." PHYSIOLOGICAL GENOMICS. 3 DEC 2002, vol. 11, no. 3, 3 December 2002 (2002-12-03), pages 219-226, XP002310950 ISSN: 1531-2267
- LEE K H: "Proteomics: a technology-driven and technology-limited discovery science" TRENDS IN BIOTECHNOLOGY, ELSEVIER, AMSTERDAM,, GB, vol. 19, no. 6, 1 June 2001 (2001-06-01), pages 217-222, XP004239791 ISSN: 0167-7799

## Description

The present invention is in the field of human genetics and relates to new methods of diagnosis of obesity, in particular morbid obesity, based on the identification of a polymorphism on the WAC gene, to method of screening potential obesity therapeutics, to pharmaceutical composition comprising said therapeutics, to transgenic non-human mammal.

Morbid obesity is a serious disease process, in which the accumulation of fatty tissue on the body becomes excessive, interferes with, or injures the other body organs, and creates (or predictably will create) serious and life-threatening health problems, which are called co-morbidities.

It is said that people are morbidly obese when their Body Mass Index (BMI = height (cm) / (mass (kg))²) is equal to or over 40.

Numerous scientific studies have established that there is a genetic predisposition to morbid obesity.

A region of the chromosome 10 has been involved in obesity (Hager J et al, Nature Genetics 1998; 20:304-338.), which has been recently confirmed in a cohort of German young obese subjects (Hinney A et al. 2000, Journal of Clinical Endocrinology and Metabolism 2000 ; 85 (8): 2962-2965), as well as in White Caucasians and in African Americans (Price RA et al., Diabetologia 2001, 44 : 363-366), and in the old order Amish (Hsueh WC et al., The Journal of Clinical Endocrinology and Metabolism 2001 ; 86(3) : 1199-1205).

The inventors thus assigned themselves the task of providing a new method of diagnosis of obesity, based on the identification of new single nucleotide polymorphism (SNP).

This objective is obtained by the following described method of diagnosis, based on the identification of a polymorphism in an intron and/or the promoter region of the WW domain-containing adapter with a coiled-coil region (WAC) gene.

Thus, the invention relates to a method for diagnosing a predisposition for obesity, and in particular morbid obesity, in a human subject which comprises determining whether there is a germline alteration at position +77 of intron 6 represented by SEQ ID NO: 1 of the WAC gene, said alteration being indicative of a predisposition to obesity.

One new SNP have been identified in the frame of the invention, located in this region, and more particularly in position +77 of intron 6 of the WAC gene, the genomic sequence of said intron is represented by SEQ ID NO: 1. This SNP shows positive association with obesity in morbidly obese subjects. The germline alteration identified therein is named SNP608.

Within the meaning of the present invention, "predisposition of obesity" is to be taken as meaning that obesity is likely to happened in subject.

Within the meaning of the present invention, "germline alteration" is to be taken as meaning that SNP 608 A>G is associated with obesity. Analysis of 275 obese trios showed that the A allele was transmitted 99 times and non transmitted 63 from heterozygous parents to affected sibs (TDT (Transmission-Disequilibrium Test ) like test, pvalue = 0.008). Furthermore, an association with obesity was observed (p=0.01) by case/control study. The A allele is more frequent in obese (74% and 75.2% respectively in morbidely and moderately obeses) than in control subjects (69.4%). In addition, in adipose tissues, expression (mRNA) levels of WAC was 1.51 fold higher for GG bearers compared to AA bearers, suggesting that genotypes of SNP 608a could modulate mRNA WAC expression. Moreover, measures of WAC expression was investigated by RT-PCR in adipose tissue from obese patients, subjected to a very low calory diet (VLCD), suggesting that WAC is regulated by the nutritional status and a relationship between WAC and leptin metabolism.

Indeed, WAC contains a WW domain, which is a conserved amino acid sequence, and which is present in proteins that seem to be involved in protein-protein interaction and has been implicated in several human diseases. Thus, WW domain conserved sequence contains a block of three tyrosine (Y) characteristic of nuclear factors and the mouse WAC homolog is colocalized with splicing factor SC35 (Xu GM, Arnaout MA (2002). WAC, a novel WW domain-containing adapter with a coiled-coil region, is colocalized with splicing factor. Genomics 79(1):87-94, suggests a potential role in pre-mRNA splicing and regulation of transcription. WW domain of WAC may regulate RNA processing, or could promote the formation of complexes with splicing factors such as SNRP70. The role of WW domain containing proteins in RNA processing has been shown in neurodegenerative diseases like Huntington (Faber PW et al. (1998) Huntingtin interacts with a family of WW domain proteins. Hum Mol Genet 7(9):1463-1474), Alzheimer's disease (Lu Pj, et al. (2002) Critical role of WW domain phosphorylation in regulating phosphoserine binding activity and Pin1 function. J Biol Chem ;277(4):2381-2384 and Minopoli G et al. (2001); The beta-amyloid precursor protein functions as a cytosolic anchoring site that prevents Fe65 nuclear translocation. J Biol Chem; 276(9):6545-6550.)

Furthermore, defects in mRNA processing (deficiency in snoRNA imprinted genes) are known to be involved in the Prader Willi syndrome which is associated with excessive appetite and progressive obesity (Wevrick R, Kerns JA, Francke U (1994); Identification of a novel paternally expressed gene in the Prader-Willi syndrome region. Hum. Mol. Genet. 3(10):1877-82).

According to one advantageous form of embodiment of the invention, the obesity is morbid obesity.

For determining a predisposition to obesity, in particular morbid obesity, in a subject, from a sample of said subject, it should be determined whether there is a mismatch between (1) a region comprising nucleotide 77 of the WAC gene genomic DNA isolated from said sample, and (2) a nucleic acid probe complementary to human wild-type region comprising nucleotide 77 of the WAC gene DNA (wherein nucleotide 77 is G), when molecules (1) and (2) are hybridized to each other to form a duplex, and said mismatch being due to the presence of the identified SNP presence of nucleotide 77.

The method according to the invention is carried out for example by amplification of WAC gene region comprising nucleotide 77 in said sample and hybridization of the amplified sequences to one or more nucleic acid probes issued from the wild-type WAC gene region comprising nucleotide 77 (as defined above) or a mutant WAC gene region comprising nucleotide 77 (in which nucleotide 77 is A).

The method may comprise determining *in situ* hybridization of the WAC gene region comprising nucleotide 77 in said sample with one or more nucleic acid probes issued from a wild-type or a mutant WAC gene region comprising nucleotide 77, as defined above.

The invention opens up a new area in the treatment and/or prevention of obesity, in particular morbid obesity, especially for patient in families where genetic susceptibility is suspected, as it gives a new target for treatment of obesity.

Thus, the invention relates also to a method for diagnosing a predisposition for obesity in a human subject, wherein the level of an expression product of the WAC gene in said sample is investigated.

The expression products according to the invention are meant to comprise mRNA or protein. Based on the following examples 2 and 3, it appears possible to diagnose a predisposition for obesity.

In one advantageous form of embodiment, the mRNA product is represented by the cDNA sequence SEQ ID NO:2 and/or SEQ ID NO:5 and/or SEQ ID NO:8.

SEQ ID NO:2 corresponds to isoform 1 of the WAC gene (accession number NM_016628),

SEQ ID NO:5 corresponds to isoform 2 of the WAC gene (accession number NM_100264), and

SEQ ID NO:8 corresponds to isoform 3 of the WAC gene (accession number NM_100486).

Each of the three isoforms of the WAC gene can be chosen to investigate the level of the expression product of the WAC gene in said sample.

In a most advantageous form of embodiment, the level of a mRNA expression product represented by the cDNA sequence SEQ ID NO:2 is investigated by the use of primers represented by the cDNA sequences SEQ ID NO:3 and SEQ ID NO:4.

In another most advantageous form of embodiment, the level of a mRNA expression product represented by the cDNA sequence SEQ ID NO:5 is investigated by the use of primers represented by the cDNA sequences SEQ ID NO:6 and SEQ ID NO:7.

In still another most advantageous form of embodiment, the level of a mRNA expression product represented by the cDNA sequence SEQ ID NO:8 is investigated by the use of primers represented by the cDNA sequences SEQ ID NO:9 and SEQ ID NO:10.

The man skilled in the art can choose all the three isoforms of the WAC gene to investigate the level of an expression product of the WAC gene.

If all the three isoforms of the WAC gene are investigated for diagnosing a predisposition to obesity in a human subject, common primers of said isoforms are used.

In another advantageous form of embodiment, the level of a mRNA expression product is investigated by the use of primers represented by the cDNA sequences SEQ ID NO:11 and SEQ ID NO:12, which are common primers of the three isoforms of the WAC gene.

Alteration of mRNA expression can be detected by any techniques known in the art. These include Northern blot analysis, RT-PCR and microarray analysis ...

In one advantageous form of embodiment, mRNA of the sample is contacted with a WAC gene probe under conditions suitable for hybridization of said probe to a RNA corresponding to said WAC gene and hybridization of said probe is determined, and the level of signal after hybridization is compared with a standard signal (which is obtained from either a non obese patient, or an obese patient).

The hybridization complex emits a signal, which may be due to the labeling of the probe, or directly of the mRNA. The different labels that may be used are well known to the person skilled in the art, and one can cite ³²P, ³³P, ³⁵S, ³H or ¹²⁵I. Non radioactive labels may be selected from ligants as biotin, avidin, streptavidin, dioxygenin, haptens, dyes, luminescent agents like radioluminescent, chemoluminescent, bioluminescent, fluorescent or phosphorescent agents.

In order to identify the SNPs mentioned in the present application, it is also possible to contact a WAC gene 5' region probe with genomic DNA isolated from said sample under conditions suitable for hybridization of said probe to said gene and hybridization of said probe is determined.

Said WAC gene 5' region probe is either a "wild-type" DNA, (i.e. the searched SNP is not present on the probe, and hybridization occurs when no SNP according to the invention is present on the DNA in the sample) or a "mutant" one (i.e. carrying the searched SNP, and hybridization only occurs if the SNP is present on the DNA in the sample).

The person skilled in the art knows the techniques to determine the allele specific probes to use in this embodiment, their lengths, or the hybridization conditions. Flanking sequences of the SNP may be used.

The person skilled in the art may use SEQ ID NO: 1 to choose the probes to use in this embodiment. A 50-300 base probe may be used in this embodiment.

In another advantageous form of embodiment, said expression product is the polypeptide encoded by the WAC gene in said sample. In particular, said polypeptide may be detected by immunoblotting or immunocytochemistry.

Said polypeptide may be the wild-type WAC polypeptide, or the mutant WAC polypeptide translated by one of the three mRNA expression products represented by SEQ ID NO:2, SEQ ID NO:5 and SEQ ID NO:8.

Antibodies specific for WAC polypeptide can be used to detect increased WAC expression. Immunological assays can be done in any convenient formats known in the art. These include Western blots, immunohistochemical assays and ELISA assays.

Antibodies may be obtained by injecting the WAC protein or WAC peptides as defined above to an animal (for example a mouse), with or without an adjuvant, and collecting the serum of said animal. Monoclonal antibodies may be obtained by immortalization of an antibody-secreting cell.

The invention relates also to a method for diagnosing a predisposition for obesity in a human subject which comprises determining whether there is a germline alteration of the promoter region at nucleotide -1218 from the start of isoforms NM_100486 and NM_016628 represented by the sequence SEQ ID NO: 13 of the WAC gene and at nucleotide -484 from the start of isoform NM_100264 represented by the sequence SEQ ID NO:14 of the WAC gene, said alteration being indicative of a predisposition to obesity.

Another new SNP have been identified in the frame of the invention, located in the promoter region of the WAC gene at nucleotide -1218 from the start of isoforms NM_100486 and NN_016628 represented by the sequence SEQ ID NO: 13 of the WAC gene and at nucleotide -484 from the start of isoform NM_100264 of said WAC gene. This SNP shows positive association with obesity. The germline alteration identified therein is named SNP829.

In a preferred form of that embodiment, the germline alteration SNP829 induces a putative binding site for Nuclear Factor-kappa B (NF-κB).

NF-κB transcription factors play an important role in the inducible regulation of a variety of genes involved in the inflammatory and proliferative responses of cells. Endogenous NF-κB activity is induced in obesity and type 2 diabetes and adipocyte NF-κB is a potential therapeutic target for obesity-linked type 2 diabetes. The presence of SNP 829 G allele induces a putative binding site for NF-κB (p50 subunit) and may enhance the circulating blood leptin level.

Example 4 presents the results obtained by the inventors that shows the correlation between the SNP829 and obesity.

Thus, the application also relates to a method for screening potential obesity drugs or therapeutics which comprises: combining (i) a WAC binding partner, (ii) a WAC polypeptide and (iii) a compound suspected of being an obesity therapeutic and determining the amount of binding of the WAC polypeptide to its binding partner and/or to (iv) a WAC regulatory element and/or to (v) a compound acting on WAC expression regulation.

In a preferred embodiment, the binding partner is a gene, a mRNA or a protein and the WAC regulatory element and the compound acting on WAC expression regulation is a protein.

In particular, as it is suspected that WAC interacts with mRNA or proteins that are involved in mRNA processing, such products can be used as binding partners. Clinical investigation in adipose tissues from obese patients showed that WAC is a gene regulated by nutritional status and be in relationship with leptin metabolism. A target of WAC gene that encodes a splicing factor could be i.e the leptin receptor. 5 forms of alternative splicing (not to mention OB receptor gene related protein or OB-RGRP), at least, have been identified for leptin receptor gene.

The application also relates to a pharmaceutical composition comprising a pharmaceutically acceptable excipient and a compound identified by a method according to the application, and to the use of a compound identified by a method according to the application for the manufacture of a drug intended to treat and/or prevent obesity, especially morbid obesity.

The application also relates to the use of a compound identified with the above-described method, or a pharmaceutical composition for the preparation of a drug intended for treatment of obesity, in particular morbid obesity.

The application also relates to the use of an antisense or sense molecule complementary to the WAC mRNA for the preparation of a drug intended for treatment of obesity, in particular morbid obesity.

The application also relates to the use of an antisense or sense molecule complementary to the WAC mRNA for the preparation of a drug intended for treatment of obesity, in particular morbid obesity.

In order to alter the activity of the WAC gene and protein, it is possible to use antisense or sense molecules or siRNA, in particular complementary to the mRNA of WAC, in a pharmaceutical composition for treating obesity, in order to alter the amount of WAC protein. The person skilled in the art is aware of the means to design antisense or sense molecules, and of the modifications that can be brought to the backbones of the molecules (phosphorothioates, methylphosphonates...).

Antisense polynucleotide sequences are useful in preventing or diminishing the expression of the WAC gene, as will be appreciated by those skilled in the art. For example, polynucleotide vectors containing all or a portion of the WAC gene may be placed under the control of a promoter in an antisense orientation and introduced into a cell. Expression of such an antisense construct within a cell will interfere with WAC transcription and/or translation.

Alternatively, a "sense" strategy may be foreseen, where a nucleic acid oligonucleotide or probe comprising part of intron 6 of the WAC gene is introduced within the cells, in order to be competitor for binding of the nuclear factors activating transcription.

The invention thus relates to a transgenic non-human mammal having integrated into its genome the nucleic acid sequence of the germline alteration of the WAC gene as previously defined, or coding sequence thereof, operatively linked to regulatory elements, wherein expression of said sequence increases the level of the WAC protein in said mammal relative to a non-transgenic mammal of the same species, or the coding sequence thereof, operatively linked to regulatory elements, wherein expression of said coding sequence increases the level of the WAC protein in said mammal relative to a non-transgenic mammal of the same species.

The WAC sequence foreseen for the preceding embodiment may be a human WAC gene sequence introduced within the genome of the animal of the invention, or the endogenous WAC gene sequence the promoter of which has been modified in order to induce overexpression. For example, the WAC coding sequence for human is known and may be found in GenBank under number NM-100486, NM-100264 or NM-016628. Other endogenous WAC genes in other animals may be identified using the homologies between sequences.

Furthermore, the invention also relates to the use of a transgenic non-human mammal whose genome comprises a disruption as previously defined of the endogenous WAC gene, as an animal model for testing links between WAC and obesity. In particular, said disruption comprises the insertion of a selectable marker sequence, and said disruption results in said non-human mammal exhibiting a defect in WAC protein as compared to a wild-type non-human mammal.

In particular, said disruption is a homozygous disruption, and said homozygous disruption results in a null mutation of the endogenous gene encoding WAC.

Expression of this gene can be increased only conditionally, using promoters that are either site- or time-specific, or even inducible. The methods to obtain animals according to the invention are known by the persons skilled in the art, and are useful in particular for testing some drugs that can be identified according to the methods of the invention.

The insertion of the construct in the genome of the transgenic animal of the invention may be performed by methods well known by the artisan in the art, and can be either random or targeted. In a few words, the person skilled in the art will construct a vector containing the sequence to insert within the genome, and a selection marker (for example the gene coding for the protein that gives resistance to neomycine), and may have it enter in the Embryonic Stem (ES) cells of an animal. The cells are then selected with the selection marker, and incorporated into an embryo, for example by microinjection into a blastocyst, that can be harvested by perfusing the uterus of pregnant females. Reimplantation of the embryo and selection of the transformed animals, followed by potential back-crossing allow to obtain such transgenic animal. To obtain a "cleaner" animal, the selection marker gene may be excised by use of a site-specific recombinase, if flanked by the correct sequences.

The mammal of the invention is preferably a mouse.

The invention also relates to the use of a mammal, as above described, as a model for studying obesity, or for testing potential anti-obesity drugs.

### Description of the figures:

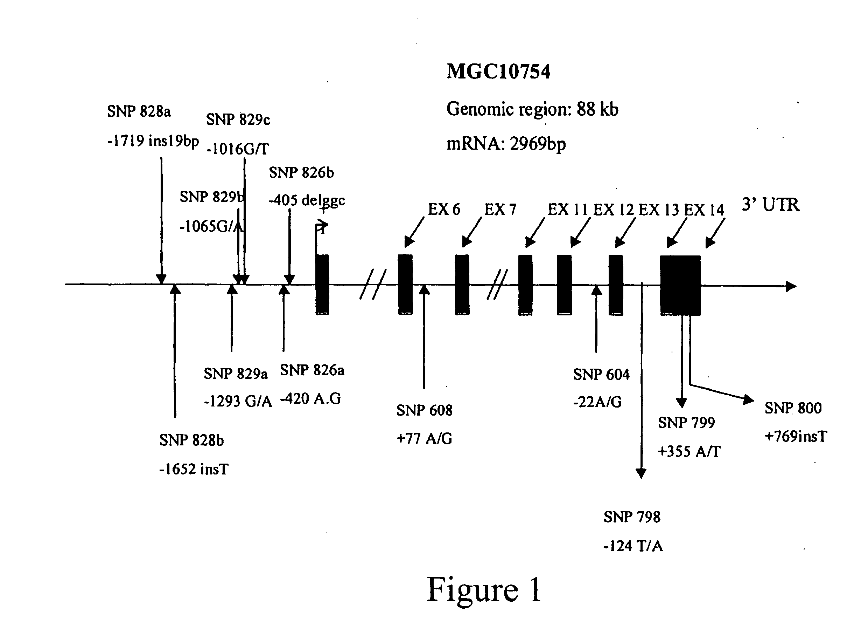

Figure 1: schematic representation of the WAC gene and of SNPs identified in the frame of the invention.

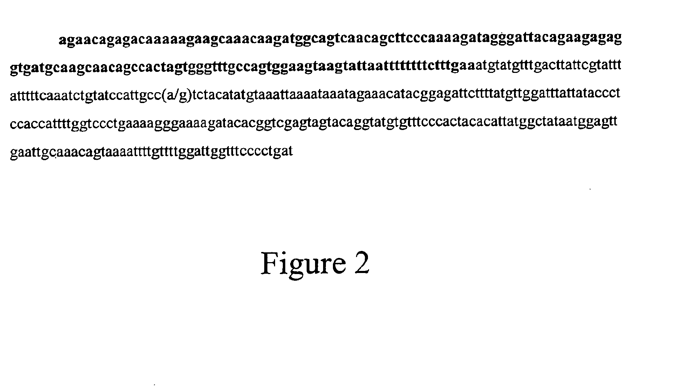

Figure 2: SEQ ID NO:1 represents end of exon 6 and intron 6 of the WAC gene including the SNP 608.

End of exon 6 is in bold.

SNP 608 of intron 6 is represented by (a/g). Figure 3: SEQ ID NO:15 represents beginning of the promoter region of the WAC gene including SNP829.

### Example 1: association between obesity and SNPs

We carried out model free multipoint analyses which revealed evidence for linkage with obesity (ASP MLS (Affected Sib-Pairs multipoint maximum LOD score ) =4.85) in a genome wide context over 15cM on chrl Op (Hager et al. Nature Genetics 1998; 20:304 338). The analysis was performed using 16 additional polymorphic markers (mean distance 1cM) in the area of linkage. Single point analyses showed a cluster of markers with a MLS higher than 1. Markers D10S1639, D10S197, and D10S600, in particular, displayed a MLS of 3.4, 3.3 and 2.54, respectively. Multipoint analysis displayed two peaks, at D10S197 (MLS = 3.2) and D10S600 (MLS = 3.4), respectively. LD (Linkage Disequilibrium) mapping strategy performed in the chromosome 10 interval of linkage with obesity allowed us to define a minimal region, enclosing two SNPs, TSC0932496 (SNP619) and TSC0618465 (SNP620) located in non coding region minimal genomic region. An haplotype defined by the G allele of SNP TSC0618465 and T allele of TSC0932496 was associated with obesity (p=0.001). This haplotype displayed increased frequency from controls (43 %), moderate and morbid obeses (51.3%) and in obeses from families contributing to the evidence of linkage on chromosome 10 (58%). Therefore, this haplotype may explain most of the evidence of linkage with obesity on chromosome 10. SNPs TSC0932496 and TSC0618465 are closed to markers D10S600 and D10S1639 (30 kb), displaying evidence of linkage and to WAC gene (160 kb).

12 SNPs of the WAC gene that we have identified, have been analysed according to our LD mapping strategy.

SNP 608a (+77 in intron 6), corresponding to nucleotide 77 of SEQ ID NO: 1, showed familial association with morbid obesity in 275 obese trios (the TDT like test, pvalue = 0.008).

The A allele was transmitted 99 times and non transmitted 63 from heterozygous parents to affected sibs. Then, SNP 608 was typed in 540 moderately obese subjects (BMI >30 kg/m2), 631 morbidly obeses (BMI>40kg/m2) and in 328 controls. An association with obesity was observed (p=0.01).

The results are represented in Table 1.

**Table 1**

| Moderately obeses (BMI>30kg/m2) OBM | Morbidely obeses (BMI>40kg/m2) SO | Controls (BMI<25kg/m2) T+ | pvalue |
|---|---|---|---|
| AA 304 (56.4) | 352 (55.8) | 157 (47.9) | SO/T+ : p=0.05 |
| AG 204 (37.7) | 230 (36.4) | 141 (43) | OBM/T+:p=0.035 |
| GG 32 (5.9) | 49 (7.8) | 30(9.1) | SO+OBM/T+ :p=0.021 |
| Fc(A)= 75.2% | Fc(A)= 74% | Fc(A)= 69.4% | SO/T+ : p=0.032 |
| Fc(G)= 24.8 % | Fc(G)= 26 % | Fc(G)= 30.6% | OBM/T+:p=0.0084 SO+OBM/T+ :p=0.0093 |

| | | | |
|---|---|---|---|
| Fc = allele frequency | | | |

### Example 2: variation of the mRNA levels

Influence of SNP 608a genotype on mRNA levels was investigated by RT-PCR in human adipose tissu. 3 adipose tissues from homozygous bearers GG of SNP 608a and 6 adipose tissues from homozygous bearers AA of SNP 608a were selected. WAC expression was compared to one reference gene (18S). Expression (mRNA) levels was 1.51 fold higher for GG bearers compared to AA bearers, suggesting that genotypes of SNP 608a could modulate mRNA WAC expression.

### Example 3: nutritional status and expression level of WAC protein

Measures of WAC expression was investigated by RT-PCR in adipose tissue from obese patients, subjected to a VLCD (48 hours and 1 month) in the hospital. The short-term protocol involved a drastic reduction of 70% daily energy intake of the 15 obese women for 48 hours. Following the two-day VLCD, their energy intake was increased to a level corresponding to their resting metabolic rate measured at day 1 (baseline). For long-term protocol, the obese women reduced one third daily energy intake (about 1200 kcal per day). None of the subjects had a familial or personal history of diabetes or were taking medication likely to affect adipocyte metabolism. Subcutaneous superficial adipose tissue samples were obtained, after an overnight fasting, by needle biopsy from the peri-umbilical area under local anesthesia (1% xylocaïne), in the control women, then at day 1 (baseline), day 3 (VLCD) and day 5 (refeeding) for the subjects taken part in short-term protocol. The same type of biopsy was performed at day 1 (baseline) and day 21 (CR) for the obese women subjected to long-term protocol. Adipose tissue specimens were immediately frozen into liquid nitrogen and stored at -80°C until analysis. Blood samples were also obtained for biochemical and hormonal evaluation.

Relative quantification of mRNA was performed with a real-time, two-step RT-PCR assay. Gene-specific primers were designed using the internet-available software "Primer3" and the sequences accessible in the GenBank database. First-strand cDNA was synthesised with 1 µg adipose tissue total RNA in a 20µl reaction volume using random hexamers as primers, according to the SuperScript first-strand cDNA synthesis system protocol (Invitrogen Life Technologies, USA). cDNA was amplified in glass capillaries using LightCycler^{®} instrument (Roche Diagnostics). PCR conditions were optimised by titration of the annealing temperature and the concentrations of primers and MgCl₂. 18S rRNA was quantified for sample normalization, using TaqMan^{®} probe approach.

The main results show that WAC expression was not modified by the short term protocol. However, in 11 subjects, the longer term VLCD, that induced a significant decrease of fat mass, WAC expression decreased of about 30% (p<0.01). In addition, WAC expression was shown to be correlated to leptin expression level (p=0.02 for Spearman correlation). These data suggest that WAC is a gene regulated by the nutritional status. In addition, the correlation with leptin expression levels might suggest a relationship between WAC and leptin metabolism in adipose tissue.

### Exemple 4: A SNP (rs2807761) from the promoter region of WAC gene modulates the leptin level

In morbidly obese subjects , SNP rs2807761 A>G was associated with variation of leptin levels. AG and GG genotypes showed higher leptin levels compared to AA genotypes (table)

**Table 2 Association studies of the SNP rs2807761 with leptin levels in morbidly obese patients**

| | | | |
|---|---|---|---|
| | N | mean | P=0.001 adjusted for gender, age and BMI |
| AA | 77 | 45,59 | |
| AG | 108 | 46,92 | |
| GG | 63 | 57,13 | |

To examine the effects of the SNP 829a on binding affinity and specificity for transcription, electrophoretic mobility shift assays (EMSAs) were performed using nuclear extracts from mouse differentiated adipocyte cells (3T3-L1) and two pairs of DNA oligonucleotide probes that differed only by the SNP. Comparisons of binding capacity of the probes containing alleles A or G-243 of SNP rs2807761, respectively, showed that 3T3-L1 nuclear proteins have a 3-fold higher affinity for the G allele variant than for the A allele of SNP rs2807761.

Computational analysis of the SNP rs2807761 flanking sequences showed that the presence of G allele of SNP rs2807761 induced a putative binding site for Nuclear Factor-kappa B (NF-κB) (p50 subunit). NF-κB transcription factors play an important role in the inducible regulation of a variety of genes involved in the inflammatory and proliferative responses of cells. Endogenous NF-κB activity is induced in obesity and type 2 diabetes and adipocyte NF-kappaB is a potential therapeutic target for obesity-linked type 2 diabetes (Ruan H, Pownall HJ, Lodish HF Troglitazone antagonizes tumor necrosis factor-alpha-induced reprogramming of adipocyte gene expression by inhibiting the transcriptional regulatory functions of NF-kappaB. J Biol Chem. 2003 Jul 25;278(30):28181-92).

## Claims

1. A method for diagnosing a predisposition for obesity in a human subject which comprises determining whether there is a germline alteration at nucleotide +77 of intron 6 represented by the sequence SEQ ID NO:1 of the WAC gene, said alteration being indicative of a predisposition to obesity with the A allele being more frequent in obese than in control subjects.

2. The method of claim 1, wherein said obesity is morbid obesity.

3. A method for diagnosing a predisposition for obesity in a human subject, from a sample from said subject, wherein the level of an expression product of the WAC gene in said sample is investigated.

4. The method of claim 3, wherein said expression product is mRNA or protein.

5. The method of claims 3 and 4, wherein the mRNA expression product is represented by the cDNA sequence SEQID N0 : 2 and/or SEQ ID NO :5 and/or SEQ ID NO : 8.

6. The method according to any of claims 3 to 5, wherein the level of a mRNA expression product represented by the cDNA sequence SEQ ID NO : 2 is investigated by the use of primers represented by the cDNA sequences SEQ ID NO : 3 and SEQ ID NO : 4.

7. The method according to any of claims 3 to 5, wherein the level of a mRNA expression product represented by the cDNA sequence SEQ ID NO :5 is investigated by the use of primers represented by the cDNA sequences SEQ ID NO : 6 and SEQ ID NO : 7.

8. The method according to any of claims 3 to 5, wherein the level of a mRNA expression product represented by the cDNA sequence SEQID N0 : 8 is investigated by the use of primers represented by the cDNA sequences SEQ ID NO: 9 and SEQ ID NO: 10.

9. The method according to any of claims 3 to 5, wherein the level of a mRNA expression product is investigated by the use of primers represented by the cDNA sequences SEQ ID NO :11 and SEQ ID NO : 12.

10. A method for diagnosing a predisposition for obesity in a human subject which comprises determining whether there is a germline alteration of the promoter region at nucleotide +148 of the WAC isoforms represented by the sequence SEQ ID NO: 13 and SEQ ID NO: 14, said alteration inducing a putative binding site for Nuclear Factor-kappa B and being indicative of a predisposition to obesity wherein said putative binding site for NF-κB (p50 subunit) is induced by the G allele.

11. A transgenic non-human mammal having integrated into its genome the nucleic acid sequence of the human WAC gene as defined in SEQ ID NO:1 comprising the A allele at nucleotide +77 of said sequence or the human WAC gene as defined in SEQ ID NO:13 or SEQ ID NO:14 comprising the G allele at nucleotide +148, operatively linked to regulatory elements, wherein expression of said sequence increases the level of the WAC protein in said mammal relative to a non-transgenic mammal of the same species.

12. The mammal of claim 11 which is a mouse.

13. Use of a mammal according to any of claims 11 to 12, as a model for studying obesity, or for testing potential anti-obesity drugs.

14. Use of a transgenic non-human mammal whose genome comprises a disruption of the endogenous WAC gene as defined in claim 1 or 10, wherein said disruption comprises the insertion of a selectable marker sequence, and wherein said disruption results in said non-human mammal exhibiting a defect in WAC protein level as compared to a wild-type non-human mammal , as a model for studying obesity, or for testing potential anti-obesity drugs.

## Patentansprüche

1. Verfahren zur Diagnose der Anfälligkeit für Fettleibigkeit bei einem Menschen, dass das Bestimmen umfasst, ob eine Veränderung der Keimbahn beim Nukleotid +77 des Introns 6 besteht, ausgedrückt durch die Sequenz SEQ ID NO:1 des WAC-Gens, wobei die Veränderung auf eine Anfälligkeit für Fettleibigkeit hinweist, wobei das A-Allel häufiger bei fettleibigen als bei kontrollierten Personen ist.

2. Verfahren nach Anspruch 1, wobei die Fettleibigkeit pathologische Fettleibigkeit ist.

3. Verfahren zur Diagnose der Anfälligkeit für Fettleibigkeit bei einem Menschen, von einer Probe von dieser Person, wobei das Niveau eines Expressionsproduktes vom WAC-Gen in der Probe ermittelt wird.

4. Verfahren nach Anspruch 3, wobei das Expressionsprodukt mRNA oder Protein ist.

5. Verfahren nach Anspruch 3 oder 4, wobei das mRNA Expressionsprodukt durch die cDNA Sequenz SEQ ID NO:2 und/oder SEQ ID NO:5 und/oder SEQ ID NO:8 ausgedrückt wird.

6. Verfahren nach einem der Ansprüche 3 bis 5, wobei das Niveau eines mRNA Expressionsproduktes ausgedrückt durch die cDNA Sequenz SEQ ID NO:2 ermittelt wird durch die Verwendung von Primers ausgedrückt durch die cDNA Sequenzen SEQ ID NO:3 und SEQ ID NO:4.

7. Verfahren nach einem der Ansprüche 3 bis 5, wobei das Niveau eines mRNA Expressionsproduktes ausgedrückt durch die cDNA Sequenz SEQ ID NO:5 ermittelt wird durch die Verwendung von Primers ausgedrückt durch die cDNA Sequenzen SEQ ID NO:6 und SEQ ID NO:7.

8. Verfahren nach einem der Ansprüche 3 bis 5, wobei das Niveau eines mRNA Expressionsproduktes ausgedrückt durch die cDNA Sequenz SEQ ID NO:8 ermittelt wird durch die Verwendung von Primers ausgedrückt durch die cDNA Sequenzen SEQ ID NO:9 und SEQ ID NO:10.

9. Verfahren nach einem der Ansprüche 3 bis 5, wobei das Niveau eines mRNA Expressionsproduktes ermittelt wird durch die Verwendung von Primers ausgedrückt durch die cDNA Sequenzen SEQ ID NO:11 und SEQ ID NO:12.

10. Verfahren zur Diagnose der Anfälligkeit für Fettleibigkeit bei einem Menschen, dass das Bestimmen umfasst, ob eine Veränderung der Keimbahn des Promoterbereichs beim Nukleotid +148 des WAC-Isoforms besteht, ausgedrückt durch die Sequenzen SEQ ID NO:13 und SEQ ID NO:14, wobei die Veränderung eine putative Bindungsstelle für Nuklear Faktor-kappa B bewirkt und auf eine Anfälligkeit für Fettleibigkeit hinweist, wobei die putative Bindungsstelle für NF-kB (p50 Untereinheit) vom G-Allel bewirkt wird.

11. Transgenes nicht menschliches Säugetier, bei dem in seinem Genom die Nukleidsäuresequenz des menschlichen WAC-Gens, wie in SEQ ID NO:1 definiert, integriert ist, umfassend das A-Allel beim Nukleotid +77 der Sequenz, oder des menschlichen WAC-Gens, wie in SEQ ID NO:13 oder SEQ ID NO:14 definiert, umfassend das G-Allel beim Nukleotid +148, wirkend verbunden mit regulatorischen Elementen, wobei die Expression der Sequenz das Niveau des WAC-Proteins im Säugetier bezüglich eines nicht transgenen Säugetiers derselben Art erhöht.

12. Säugetier von Anspruch 11, dass eine Maus ist.

13. Verwendung eines Säugetiers nach einem der Ansprüche 11 bis 12, als ein Modell zum Untersuchen von Fettleibigkeit, oder zum Testen von potentiellen Anti-Fettleibigkeit-Medikamenten.

14. Verwendung eines transgenen nicht menschlichen Säugetiers, dessen Genom eine Unterbrechung des endogenen WAC-Gens, wie in Anspruch 1 oder 10 definiert, umfasst, wobei die Unterbrechung die Insertion einer wählbaren Marker-Sequenz umfasst, und wobei die Unterbrechung im nicht menschlichen Säugetier bewirkt, einen Defekt im WAC-Proteinniveau im Vergleich zu einem nicht menschlichen Säugetier des wilden Typs aufzuzeigen, als ein Modell zum Untersuchen von Fettleibigkeit, oder zum Testen von potentiellen Anti-Fettleibigkeit-Medikamenten.

## Revendications

1. Procédé pour diagnostiquer une prédisposition à l'obésité chez un sujet humain, qui comprend la détermination de l'existence d'une altération germinale au nucléotide +77 de l'intron 6 représenté par la SEQ ID NO : 1 du gène WAC, ladite altération étant indicative d'une prédisposition à l'obésité, l'allèle A étant plus fréquent chez les sujets obèses que chez les sujets témoins.

2. Procédé selon la revendication 1, dans lequel ladite obésité est une obésité morbide.

3. Procédé pour diagnostiquer une prédisposition à l'obésité chez un sujet humain, à partir d'un échantillon dudit sujet, dans lequel le taux d'un produit d'expression du gène WAC dans ledit échantillon est étudié.

4. Procédé selon la revendication 3, dans lequel ledit produit d'expression est un ARNm ou une protéine.

5. Procédé selon les revendications 3 et 4, dans lequel le produit d'expression d'ARNm est représenté par la séquence d'ADNc de la SEQ ID NO : 2 et/ou de la SEQ ID NO : 5 et/ou de la SEQ ID NO : 8.

6. Procédé selon l'une quelconque des revendications 3 à 5, dans lequel le taux d'un produit d'expression d'ARNm représenté par la séquence d'ADNc de la SEQ ID NO : 2 est étudié par l'utilisation d'amorces représentées par les séquences d'ADNc de la SEQ ID NO : 3 et de la SEQ ID NO : 4.

7. Procédé selon l'une quelconque des revendications 3 à 5, dans lequel le taux d'un produit d'expression d'ARNm représenté par la séquence d'ADNc de la SEQ ID NO : 5 est étudié par l'utilisation d'amorces représentées par les séquences d'ADNc de la SEQ ID NO : 6 et de la SEQ ID NO : 7.

8. Procédé selon l'une quelconque des revendications 3 à 5, dans lequel le taux d'un produit d'expression d'ARNm représenté par la séquence d'ADNc de la SEQ ID NO : 8 est étudié par l'utilisation d'amorces représentées par les séquences d'ADNc de la SEQ ID NO : 9 et de la SEQ ID NO : 10.

9. Procédé selon l'une quelconque des revendications 3 à 5, dans lequel le taux d'un produit d'expression d'ARNm est étudié par l'utilisation d'amorces représentées par les séquences d'ADNc de la SEQ ID NO : 11 et de la SEQ ID NO : 12.

10. Procédé pour diagnostiquer une prédisposition à l'obésité chez un sujet humain, qui comprend la détermination de l'existence d'une altération germinale de la région de promoteur au niveau du nucléotide +148 des isoformes de WAC représentées par les séquences de la SEQ ID NO : 13 et de la SEQ ID NO : 14, ladite altération induisant un site de liaison putatif pour le facteur nucléaire kappa-B et étant indicative d'une prédisposition à l'obésité, dans lequel ledit site de liaison putatif pour NF-κB (sous-unité p50) est induit par l'allèle G.

11. Mammifère non-humain transgénique dans le génome duquel est intégré la séquence d'acide nucléique du gène WAC humain, telle que définie dans la SEQ ID NO : 1, comprenant l'allèle A au niveau du nucléotide +77 de ladite séquence, ou du gène WAC humain, telle que définie dans la SEQ ID NO : 13 ou la SEQ ID NO : 14, comprenant l'allèle G au niveau du nucléotide +148, liée de façon opérable à des éléments régulateurs, dans lequel l'expression de ladite séquence augmente le taux de la protéine WAC dans ledit mammifère, par rapport à un animal non-transgénique de la même espèce.

12. Mammifère selon la revendication 11, qui est une souris.

13. Utilisation d'un mammifère selon l'une quelconque des revendications 11 et 12, en tant que modèle pour étudier l'obésité, ou pour tester des médicaments anti-obésité potentiels.

14. Utilisation d'un mammifère non-humain transgénique dont le génome comprend une interruption du gène WAC endogène, telle que définie dans la revendication 1 à 10, dans laquelle ladite interruption comprend l'insertion d'une séquence de marqueur sélectionnable, et dans laquelle ladite interruption a pour résultat que ledit mammifère non-humain présente un défaut du taux de protéine WAC par comparaison avec un mammifère non-humain de type sauvage, en tant que modèle pour étudier l'obésité, ou pour tester des médicaments anti-obésité potentiels.


### SEQUENCE LISTING

<110> CENTRE NATIONAL DE LA RECHERCHE SCIENTIFIQUE (CNRS)
   <120> METHOD OF DIAGNOSIS OF OBESITY
   <130> D20472
   <150> EP 03/292 202
   <151> 2003-09-08
   <160> 14
   <170> PatentIn version 3.2
<210> 1
   <211> 281
   <212> DNA
   <213> Homo sapiens
<220>
   <223> SNP 608 in position 77
<400> 1
   gtaagtatta attttttttc tttgaaatgt atgtttgact tattcgtatt tatttttcaa     60
   atctgtatcc attgccrtct acatatgtaa attaaaataa atagaaacat acggagattc    120
   ttttatgttg gatttattat accctccacc attttggtcc ctgaaaaggg aaaagataca    180
   cggtcgagta gtacaggtat gtgtttccca ctacacatta tggctataat ggagttgaat    240
   tgcaaacagt aaaattttgt tttggattgg tttcccctga t                        281
<210> 2
   <211> 2967
   <212> DNA
   <213> Homo sapiens
<220>
   <223> Isoform 1 - accession number NM_016628
<400> 2
   cgccgcctgc gcgcccgccc gcctttcgcg gccgctctcc cccctccccg acacacactc     60
   acaggccggg cattgatggt aatgtatgcg aggaaacagc agagactcag tgatggctgt    120
   cacgaccgga ggggggactc gcagccttac caggcactta agtattcatc gaagagtcac    180
   cccagtagcg gtgatcacag acatgaaaag atgcgagacg ccggagatcc ttcaccacca    240
   aataaaatgt tgcggagatc tgatagtcct gaaaacaaat acagtgacag cacaggtcac    300
   agtaaggcca aaaatgtgca tactcacaga gttagagaga gggatggtgg gaccagttac    360
   tctccacaag aaaattcaca caaccacagt gctcttcata gttcaaattc acattcttct    420
   aatccaagca ataacccaag caaaacttca gatgcacctt atgattctgc agatgactgg    480
   tctgagcata ttagctcttc tgggaaaaag tactactaca attgtcgaac agaagtttca    540
   caatgggaaa aaccaaaaga gtggcttgaa agagaacaga gacaaaaaga agcaaacaag    600
   atggcagtca acagcttccc aaaagatagg gattacagaa gagaggtgat gcaagcaaca    660
   gccactagtg ggtttgccag tggaatggaa gacaagcatt ccagtgatgc cagtagtttg    720
   ctcccacaga atattttgtc tcaaacaagc agacacaatg acagagacta cagactgcca    780
   agagcagaga ctcacagtag ttctacgcca gtacagcacc ccatcaaacc agtggttcat    840
   ccaactgcta ccccaagcac tgttccttct agtccattta cgctacagtc tgatcaccag    900
   ccaaagaaat catttgatgc taatggagca tctactttat caaaactgcc tacacccaca    960
   tcttctgtcc ctgcacagaa aacagaaaga aaagaatcta catcaggaga caaacccgta   1020
   tcacattctt gcacaactcc ttccacgtct tctgcctctg gactgaaccc cacatctgca   1080
   cctccaacat ctgcttcagc ggtccctgtt tctcctgttc cacagtcgcc aatacctccc   1140
   ttacttcagg acccaaatct tcttagacaa ttgcttcctg ctttgcaagc cacgctgcag   1200
   cttaataatt ctaatgtgga catatctaaa ataaatgaag ttcttacagc agctgtgaca   1260
   caagcctcac tgcagtctat aattcataag tttcttactg ctggaccatc tgctttcaac   1320
   ataacgtctc tgatttctca agctgctcag ctctctacac aagcccagcc atctaatcag   1380
   tctccgatgt ctttaacatc tgatgcgtca tccccaagat catatgtttc tccaagaata   1440
   agcacacctc aaactaacac agtccctatc aaacctttga tcagtactcc tcctgtttca   1500
   tcacagccaa aggttagtac tccagtagtt aagcaaggac cagtgtcaca gtcagccaca   1560
   cagcagcctg taactgctga caagcagcaa ggtcatgaac ctgtctctcc tcgaagtctt   1620
   cagcgctcaa gtagccagag aagtccatca cctggtccca atcatacttc taatagtagt   1680
   aatgcatcaa atgcaacagt tgtaccacag aattcttctg cccgatccac gtgttcatta   1740
   acgcctgcac tagcagcaca cttcagtgaa aatctcataa aacacgttca aggatggcct   1800
   gcagatcatg cagagaagca ggcatcaaga ttacgcgaag aagcgcataa catgggaact   1860
   attcacatgt ccgaaatttg tactgaatta aaaaatttaa gatctttagt ccgagtatgt   1920
   gaaattcaag caactttgcg agagcaaagg atactatttt tgagacaaca aattaaggaa   1980
   cttgaaaagc taaaaaatca gaattccttc atggtgtgaa gatgtgaata attgcacatg   2040
   gttttgagaa caggaactgt aaatctgttg cccaatctta acatttttga gctgcattta   2100
   agtagacttt ggaccgttaa gctgggcaaa ggaaatgaca aggggacggg gtctgtgaga   2160
   gtcaattcag gggaaagata caagattgat ttgtaaaacc cttgaaatgt agatttcttg   2220
   tagatgtatc cttcacgttg taaatatgtt ttgtagagtg aagccatggg aagccatgtg   2280
   taacagagct tagacatcca aaactaatca atgctgaggt ggctaaatac ctagcctttt   2340
   acatgtaaac ctgtctgcaa aattagcttt tttaaaaaaa aaaaaaaaaa aattgggggg   2400
   gttaatttat cattcagaaa tcttgcattt tcaaaaattc agtgcaagcg ccaggcgatt   2460
   tgtgtctaag gatacgattt tgaaccatat gggcagtgta caaaatatga aacaactgtt   2520
   tccacacttg cacctgatca agagcagtgc ttctccattt gttttgcaga gaaatgtttt   2580
   tcatttcccg tgtgtttcca tttccttctg aaattctgat tttatccatt tttttaaggc   2640
   tcctctttat ctcctttctt aaggcactgt tgctatggca cttttctata accttttcat   2700
   tcctgtgtac agtagcttaa aattgcagtg attgagcata acctacttgt ttgtataaat   2760
   tattgaaatc catttgcacc ctgtaagaat ggacttaaaa gtactgctgg acaggcatgt   2820
   gtgctcaaag tacattgatt gctcaaatat aaggaaatgg cccaatgaac gtggttgtgg   2880
   gaggggaaag aggaaacaga gctagtcaga tgtgaattgt atctgttgta ataaacatgt   2940
   taaaacaaac aaaaaaaaaa aaaaaaa                                        2967
<210> 3
   <211> 20
   <212> DNA
   <213> Homo sapiens
<220>
   <223> Primer (Waciso1F)
<400> 3
   atggtaatgt atgcgaggaa 20
<210> 4
   <211> 20
   <212> DNA
   <213> Homo sapiens
<220>
   <223> Primer (Waciso1R)
<400> 4
   cttgtggaga gtaactggtc 20
<210> 5
   <211> 3088
   <212> DNA
   <213> Homo sapiens
<220>
   <223> Isoform 2 - accession number NM_100264
<400> 5
   ccttatttag tccgcgatgg cttccctcgc gccccaccgt cctcttccgg aaggcggctc      60
   cctccctgcg cagcccggag cccctgagat cagcctcgag caggcgcccg agcgagacta     120
   tccctaaacg ggaacggcgg tggccgactc gcgagtgagg aaaagaagga aagggcagac     180
   tggtcgcgaa gagaagatcc aggcctcaga ggaggagaaa ggccggagcc agccgagctg     240
   tcacgaccgg aggggggact cgcagcctta ccaggcactt aagtattcat cgaagagtca     300
   ccccagtagc ggtgatcaca gacatgaaaa gatgcgagac gccggagatc cttcaccacc     360
   aaataaaatg ttgcggagat ctgatagtcc tgaaaacaaa tacagtgaca gcacaggtca     420
   cagtaaggcc aaaaatgtgc atactcacag agttagagag agggatggtg ggaccagtta     480
   ctctccacaa gaaaattcac acaaccacag tgctcttcat agttcaaatt cacattcttc     540
   taatccaagc aataacccaa gcaaaacttc agatgcacct tatgattctg cagatgactg     600
   gtctgagcat attagctctt ctgggaaaaa gtactactac aattgtcgaa cagaagtttc     660
   acaatgggaa aaaccaaaag agtggcttga aagagaacag agacaaaaag aagcaaacaa     720
   gatggcagtc aacagcttcc caaaagatag ggattacaga agagaggtga tgcaagcaac     780
   agccactagt gggtttgcca gtggaatgga agacaagcat tccagtgatg ccagtagttt     840
   gctcccacag aatattttgt ctcaaacaag cagacacaat gacagagact acagactgcc     900
   aagagcagag actcacagta gttctacgcc agtacagcac cccatcaaac cagtggttca     960
   tccaactgct accccaagca ctgttccttc tagtccattt acgctacagt ctgatcacca    1020
   gccaaagaaa tcatttgatg ctaatggagc atctacttta tcaaaactgc ctacacccac    1080
   atcttctgtc cctgcacaga aaacagaaag aaaagaatct acatcaggag acaaacccgt    1140
   atcacattct tgcacaactc cttccacgtc ttctgcctct ggactgaacc ccacatctgc    1200
   acctccaaca tctgcttcag cggtccctgt ttctcctgtt ccacagtcgc caatacctcc    1260
   cttacttcag gacccaaatc ttcttagaca attgcttcct gctttgcaag ccacgctgca    1320
   gcttaataat tctaatgtgg acatatctaa aataaatgaa gttcttacag cagctgtgac    1380
   acaagcctca ctgcagtcta taattcataa gtttcttact gctggaccat ctgctttcaa    1440
   cataacgtct ctgatttctc aagctgctca gctctctaca caagcccagc catctaatca    1500
   gtctccgatg tctttaacat ctgatgcgtc atccccaaga tcatatgttt ctccaagaat    1560
   aagcacacct caaactaaca cagtccctat caaacctttg atcagtactc ctcctgtttc    1620
   atcacagcca aaggttagta ctccagtagt taagcaagga ccagtgtcac agtcagccac    1680
   acagcagcct gtaactgctg acaagcagca aggtcatgaa cctgtctctc ctcgaagtct    1740
   tcagcgctca agtagccaga gaagtccatc acctggtccc aatcatactt ctaatagtag    1800
   taatgcatca aatgcaacag ttgtaccaca gaattcttct gcccgatcca cgtgttcatt    1860
   aacgcctgca ctagcagcac acttcagtga aaatctcata aaacacgttc aaggatggcc    1920
   tgcagatcat gcagagaagc aggcatcaag attacgcgaa gaagcgcata acatgggaac    1980
   tattcacatg tccgaaattt gtactgaatt aaaaaattta agatctttag tccgagtatg    2040
   tgaaattcaa gcaactttgc gagagcaaag gatactattt ttgagacaac aaattaagga    2100
   acttgaaaag ctaaaaaatc agaattcctt catggtgtga agatgtgaat aattgcacat    2160
   ggttttgaga acaggaactg taaatctgtt gcccaatctt aacatttttg agctgcattt     2220
   aagtagactt tggaccgtta agctgggcaa aggaaatgac aaggggacgg ggtctgtgag    2280
   agtcaattca ggggaaagat acaagattga tttgtaaaac ccttgaaatg tagatttctt    2340
   gtagatgtat ccttcacgtt gtaaatatgt tttgtagagt gaagccatgg gaagccatgt    2400
   gtaacagagc ttagacatcc aaaactaatc aatgctgagg tggctaaata cctagccttt    2460
   tacatgtaaa cctgtctgca aaattagctt ttttaaaaaa aaaaaaaaaa aaattggggg    2520
   ggttaattta tcattcagaa atcttgcatt ttcaaaaatt cagtgcaagc gccaggcgat    2580
   ttgtgtctaa ggatacgatt ttgaaccata tgggcagtgt acaaaatatg aaacaactgt    2640
   ttccacactt gcacctgatc aagagcagtg cttctccatt tgttttgcag agaaatgttt    2700
   ttcatttccc gtgtgtttcc atttccttct gaaattctga ttttatccat ttttttaagg    2760
   ctcctcttta tctcctttct taaggcactg ttgctatggc acttttctat aaccttttca     2820
   ttcctgtgta cagtagctta aaattgcagt gattgagcat aacctacttg tttgtataaa    2880
   ttattgaaat ccatttgcac cctgtaagaa tggacttaaa agtactgctg gacaggcatg    2940
   tgtgctcaaa gtacattgat tgctcaaata taaggaaatg gcccaatgaa cgtggttgtg    3000
   ggaggggaaa gaggaaacag agctagtcag atgtgaattg tatctgttgt aataaacatg    3060
   ttaaaacaaa caaaaaaaaa aaaaaaaa                                       3088
<210> 6
   <211> 20
   <212> DNA
   <213> Homo sapiens
<220>
   <223> Primer (Waciso2F)
<400> 6
   ccgagcgaga ctatccctaa 20
<210> 7
   <211> 20
   <212> DNA
   <213> Homo sapiens
<220>
   <223> Primer (Waciso2R)
<400> 7
   agagcactgt ggttgtgtga 20
<210> 8
   <211> 2647
   <212> DNA
   <213> Homo sapiens
<220>
   <223> Isoform 3 - accession number NM_100486
<400> 8
   cgccgcctgc gcgcccgccc gcctttcgcg gccgctctcc cccctccccg acacacactc      60
   acaggccggg cattgatggt aatgtatgcg aggaaacagc agagactcag tgatggctgt     120
   cacgaccgga ggggggactc gcagccttac caggcactta agtattcatc gaagagtcac      180
   cccagtagcg gtgatcacag acatgaaaag atgcgagacg ccggagatcc ttcaccacca     240
   aataaaatgt tgcggagatc tgatagtcct gaaaacaaat acagtgacag cacaggtcac     300
   agtaaggcca aaaatgtgca tactcacaga gttagagaga gggatggtgg gaccagttac     360
   tctccacaag aaaattcaca caaccacagt gctcttcata gttcaaattc acattcttct     420
   aatccaagca ataacccaag caaaacttca gatgcacctt atgattctgc agatgactgg     480
   tctgagcata ttagctcttc tgggaaaaag tactactaca attgtcgaac agaagtttca     540
   caatgggaaa aaccaaaaga gtggcttgaa agagaacaga gacaaaaaga agcaaacaag     600
   atggcagtca acagcttccc aaaagatagg gattacagaa gagaggtgat gcaagcaaca     660
   gccactagtg ggtttgccag tggaaaatct acatcaggag acaaacccgt atcacattct     720
   tgcacaactc cttccacgtc ttctgcctct ggactgaacc ccacatctgc acctccaaca     780
   tctgcttcag cggtccctgt ttctcctgtt ccacagtcgc caatacctcc cttacttcag     840
   gacccaaatc ttcttagaca attgcttcct gctttgcaag ccacgctgca gcttaataat     900
   tctaatgtgg acatatctaa aataaatgaa gttcttacag cagctgtgac acaagcctca     960
   ctgcagtcta taattcataa gtttcttact gctggaccat ctgctttcaa cataacgtct    1020
   ctgatttctc aagctgctca gctctctaca caagcccagc catctaatca gtctccgatg    1080
   tctttaacat ctgatgcgtc atccccaaga tcatatgttt ctccaagaat aagcacacct    1140
   caaactaaca cagtccctat caaacctttg atcagtactc ctcctgtttc atcacagcca    1200
   aaggttagta ctccagtagt taagcaagga ccagtgtcac agtcagccac acagcagcct    1260
   gtaactgctg acaagcagca aggtcatgaa cctgtctctc ctcgaagtct tcagcgctca    1320
   agtagccaga gaagtccatc acctggtccc aatcatactt ctaatagtag taatgcatca    1380
   aatgcaacag ttgtaccaca gaattcttct gcccgatcca cgtgttcatt aacgcctgca    1440
   ctagcagcac acttcagtga aaatctcata aaacacgttc aaggatggcc tgcagatcat    1500
   gcagagaagc aggcatcaag attacgcgaa gaagcgcata acatgggaac tattcacatg    1560
   tccgaaattt gtactgaatt aaaaaattta agatctttag tccgagtatg tgaaattcaa    1620
   gcaactttgc gagagcaaag gatactattt ttgagacaac aaattaagga acttgaaaag    1680
   ctaaaaaatc agaattcctt catggtgtga agatgtgaat aattgcacat ggttttgaga    1740
   acaggaactg taaatctgtt gcccaatctt aacatttttg agctgcattt aagtagactt    1800
   tggaccgtta agctgggcaa aggaaatgac aaggggacgg ggtctgtgag agtcaattca    1860
   ggggaaagat acaagattga tttgtaaaac ccttgaaatg tagatttctt gtagatgtat    1920
   ccttcacgtt gtaaatatgt tttgtagagt gaagccatgg gaagccatgt gtaacagagc    1980
   ttagacatcc aaaactaatc aatgctgagg tggctaaata cctagccttt tacatgtaaa    2040
   cctgtctgca aaattagctt ttttaaaaaa aaaaaaaaaa aaattggggg ggttaattta    2100
   tcattcagaa atcttgcatt ttcaaaaatt cagtgcaagc gccaggcgat ttgtgtctaa    2160
   ggatacgatt ttgaaccata tgggcagtgt acaaaatatg aaacaactgt ttccacactt    2220
   gcacctgatc aagagcagtg cttctccatt tgttttgcag agaaatgttt ttcatttccc    2280
   gtgtgtttcc atttccttct gaaattctga ttttatccat ttttttaagg ctcctcttta    2340
   tctcctttct taaggcactg ttgctatggc acttttctat aaccttttca ttcctgtgta    2400
   cagtagctta aaattgcagt gattgagcat aacctacttg tttgtataaa ttattgaaat    2460
   ccatttgcac cctgttaaga atggacttaa aagtactgct ggacaggcat gtgtgctcaa    2520
   agtacattga ttgctcaaat ataaggaaat ggcccaatga acgtggttgt gggaggggaa    2580
   agaggaaaca gagctagtca gatgtgaatt gtatctgttg taataaacat gttaaaacaa    2640
   acaaaaa                                                              2647
<210> 9
   <211> 25
   <212> DNA
   <213> Homo sapiens
<220>
   <223> Primer (Waciso3F)
<400> 9
   ttgccagtgg aaaatctaca tcagg 25
<210> 10
   <211> 20
   <212> DNA
   <213> Homo sapiens
<220>
   <223> Primer (Waciso3R)
<400> 10
   ggaacaggag aaacagggac 20
<210> 11
   <211> 19
   <212> DNA
   <213> Homo sapiens
<220>
   <223> Forward primer - common for the 3 isoforms
<400> 11
   ggactcgcag ccttaccag 19
<210> 12
   <211> 21
   <212> DNA
   <213> Homo sapiens
<220>
   <223> Reverse primer - common for the 3 isoforms
<400> 12
   gttttgcttg ggttattgct t 21
<210> 13
   <211> 1481
   <212> DNA
   <213> Homo sapiens
<220>
   <223> SNP 829 in the promoter region of the isoforms 1 and 3 (respectively NM_016628 and NM_100486)
<400> 13
   ctcagactgg acgacagcgc gagacctgtc tcaaaaaaaa ataacaaggg gtagggtggg      60
   gcagtgagat ggttcagcgt tgttcccaag gaactcatct ccctaaattt ccagtctcaa     120
   gcgccgcttt cattccctgc cccgagcrgc gctccccgct ccgtgcacgc atctccgctg     180
   agggccgccc tgacgcacgg ccttctgcct tgaacacaga acccctcagc ccttcctatt     240
   agccgtttct ggccgggact gcacgcgctc cttcctgcct cggggcgcag ggtgagactt     300
   acgtctaaag ggcgcccggc gagctcgcag cagcccctgc cccgcggagg gccggtggcg     360
   cgcggcgtcc ggtgaggggc agcggcccgg gctcggttcc ggcttccagc agcggcccga     420
   aggcgccgga gcacgctccc cgctccccag aacgcagctt ccgcggctgc agcccaggca     480
   gctcttcggg aagcgtccgc ccgtggtctc gccagggggt ttccgacctc cccgcccgac     540
   agcgcagggg cgggggccgc ggccggagag ctcgcccgag agcaccccct cccgctgcgc     600
   ctctgggcgc acgcgcagcc gctgcagcct cccttattta gtccgcgatg gcttccctcg     660
   cgccccaccg tcctcttccg gaaggcggct ccctccctgc gcagcccgga gcccctgaga     720
   tcagcctcga gcaggcgccc gagcgagact atccctaaac gggaacggcg gtggccgact     780
   cgcgagtgag gaaaagaagg aaagggcaga ctggtcgcga agagaagatc caggcctcag     840
   aggaggagaa aggccggagc cagccgaggt ttgccgaggg cggtgttccg gacccgcgcg     900
   gtgcggggag gaaggccgag ggtgggagag gaggggcccg gcggaaactg ccgaggtttc     960
   ccgaaggcgg cagcgtccga gttgcccgga tgtagttggt ggagcggcag cggcggcacc    1020
   agcggcggcg gcggcggcgg gaggaggagg aggagaagaa ggaccaggcg gcggcagcag    1080
   cggcggcggc ggggggagga ggggaggagg cggcggagca ggaggaggag aaggcggagg    1140
   aggcagtcgc tctccgcggg gctgagccgg acgcgtcgtc ttgcccccct ccccccggtt     1200
   cgcggtgccg ccgtgtagtt ggcgccgctg ccccggctga gagtgagcgt ggtgtcgacg    1260
   gagggagatg gcccgggagc gccggcgcca gtaactggga gctgatgaga gtcgccgagg    1320
   gcgcgccggg cccaggtgcc ggggctgccc gccgcccgcc gccgccgccg cctgcgcgcc    1380
   cgcccgcctt tcgcggccgc tctcccccct ccccgacaca cactcacagg ccgggcattg    1440
   atggtaatgt atgcgaggaa acagcagaga ctcagtgatg g                         1481
<210> 14
   <211> 868
   <212> DNA
   <213> Homo sapiens
<220>
   <223> SNP 829 in the promoter region of the isoform 2 (NM_100264)
<400> 14
   ctcagactgg acgacagcgc gagacctgtc tcaaaaaaaa ataacaaggg gtagggtggg      60
   gcagtgagat ggttcagcgt tgttcccaag gaactcatct ccctaaattt ccagtctcaa     120
   gcgccgcttt cattccctgc cccgagcrgc gctccccgct ccgtgcacgc atctccgctg     180
   agggccgccc tgacgcacgg ccttctgcct tgaacacaga acccctcagc ccttcctatt     240
   agccgtttct ggccgggact gcacgcgctc cttcctgcct cggggcgcag ggtgagactt     300
   acgtctaaag ggcgcccggc gagctcgcag cagcccctgc cccgcggagg gccggtggcg     360
   cgcggcgtcc ggtgaggggc agcggcccgg gctcggttcc ggcttccagc agcggcccga     420
   aggcgccgga gcacgctccc cgctccccag aacgcagctt ccgcggctgc agcccaggca     480
   gctcttcggg aagcgtccgc ccgtggtctc gccagggggt ttccgacctc cccgcccgac     540
   agcgcagggg cgggggccgc ggccggagag ctcgcccgag agcaccccct cccgctgcgc     600
   ctctgggcgc acgcgcagcc gctgcagcct cccttattta gtccgcgatg gcttccctcg     660
   cgccccaccg tcctcttccg gaaggcggct ccctccctgc gcagcccgga gcccctgaga     720
   tcagcctcga gcaggcgccc gagcgagact atccctaaac gggaacggcg gtggccgact     780
   cgcgagtgag gaaaagaagg aaagggcaga ctggtcgcga agagaagatc caggcctcag     840
   aggaggagaa aggccggagc cagccgag                                        868